# EUROPEAN PATENT APPLICATION

(11) **EP 2 886 116 A1**
(43) Date of publication of application: **24.06.2015**
(21) Application number: 13306768.6
(22) Date of filing: 18.12.2013
(51) Int. Cl.: A61K 31/343, A61P 25/00

(54) **Benzodioxol derivatives for use in the treatment of attention deficit and/or hyperactivity**

(71) Applicant: Biocodex, 94250 Gentilly (FR)
(72) Inventor: Verleye, Marc, 60190 Remy (FR); Riban, Véronique, 60200 Compiegne (FR); Le Guern, Marie-Emmanuelle, 60200 Compiegne (FR)
(74) Representative: Vial, Lionel

(57) **Abstract**

The present invention relates to a compound of the following formula (I): 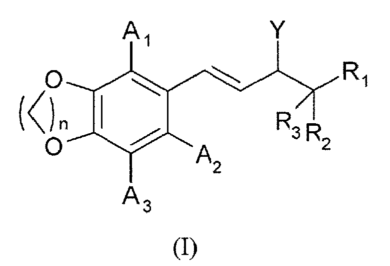 or a pharmaceutically acceptable salt thereof,
for use in the prevention or treatment of attention deficit and/or hyperactivity in an individual.

## Description

### Field of the invention

The present invention relates to method and compositions for treating attention deficit and/or hyperactivity.

### Technical background

Attention deficit hyperactivity disorder (ADHD) as defined by the Diagnostic and Statistical Manual of Mental Disorders (DSM), which may also be referred to as hyperkinetic disorder as defined by the International Classification of Diseases (ICD), is a psychiatric disorder in which there are significant problems of impulsivity, hyperactivity and short attention span, that are not appropriate for a person's age.

This disorder affects about 6 to 7 percent of children when diagnosed via the DSM-IV criteria and 1 to 2 percent when diagnosed via the ICD-10 criteria. ADHD is approximately three times more frequent in boys than in girls. In school-aged children the lack of focus resulting from ADHD often leads to poor school performance.

ADHD management usually involves some combination of counseling, lifestyle changes, and medications. Among medications, the drug of choice is methylphenidate (Ritalin®), a central nervous system stimulant. However, methylphenidate has potential to induce drug dependency or long-term changes in brain development (Lagace et al. (2006) Biol. Psychiatry 60:1121-1130).

Accordingly, there is a need for other drugs to manage ADHD.

Stiripentol (Diacomit, 1-penten-3-ol,1-(1,3-benzodioxol)-4,4-dimethyl or 4-dimethyl-1-[3,4-methylenedioxy-3,4)-phenyl]-1-penten-3-ol) is a racemic allylic alcohol that is structurally unrelated to other antiepileptic drugs.

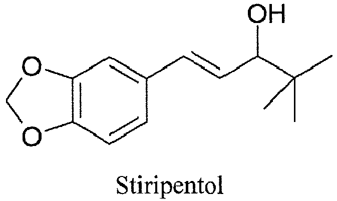

Stiripentol has shown anticonvulsant activity in several animal models but its spectrum of clinical activity is relatively narrow. The efficacy of Stiripentol, as an add-on therapy to valproate and clobazam, in controlling epilepsy seizures in children presenting with Dravet syndrome, was demonstrated in two randomized, double-blind, placebo-controlled studies. The primary efficacy endpoint for both studies was responder rate. A responder was defined as a patient who experienced a ≥50% decrease in the frequency of generalized clonic or tonic-clonic seizures during the double-blind treatment period compared to baseline (Chiron (2000) Lancet 356:1638). Stiripentol was granted a European Marketing Authorisation for this indication.

### Summary of the invention

The present invention arises from the unexpected finding, by the inventors, that Stiripentol is useful in a rat model of hyperactivity.

The present invention thus relates to a compound of the following formula (I): 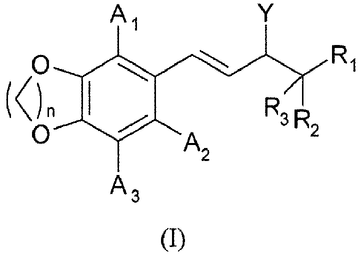 wherein:
- n represents 1 or 2,
- A₁, A₂ and A₃, which may be identical or different, represent a hydrogen atom, a halogen atom or a linear or branched alkyl group having from 1 to 4 carbon atoms,
- R₁, R₂ and R₃ represent independently a hydrogen atom or a linear or branched alkyl group having from 1 to 4 carbon atoms, and
- Y represents -OH, =O or -SH;
or a pharmaceutically acceptable salt thereof,
for use in the prevention or treatment of attention deficit and/or hyperactivity and/or impulsivity in an individual.

The present invention also relates to a method for the prevention or treatment of of attention deficit and/or hyperactivity and/or impulsivity in an individual, comprising administering the individual a prophylactically or therapeutically effective quantity of at least one compound of formula (I) as defined above.

The present invention also relates to the use of at least one compound of formula (I) as defined above for the manufacture of a medicament intended for the prevention or treatment of attention deficit and/or hyperactivity and/or impulsivity in an individual.

In an embodiment of the compound or pharmaceutically acceptable salt thereof for its use as defined above, the method as defined above or the use as defined above, the compound of formula (I) is in combination, or is combined, with at least one additional compound intended for preventing or treating attention deficit and/or hyperactivity and/or impulsivity.

In another embodiment of the compound or pharmaceutically acceptable salt thereof for its use as defined above, the method as defined above or the use as defined above, the compound of formula (I) is not in combination, or combined, with another prophylactically or therapeutically active compound, such as a psycho-stimulant (also called a stimulant), in particular methylphenidate or dexamfetamine, an Al adenosine receptor agonist, an A2A adenosine receptor agonist, or a lithium salt.

The present invention also relates to a pharmaceutical composition, comprising as active substance, at least one compound of formula (I) as defined above, or a pharmaceutically acceptable salt thereof, for use in the prevention or treatment of attention deficit and/or hyperactivity and/or impulsivity in an individual.

In an embodiment of the invention, the pharmaceutical composition for use as defined above further comprises at least one additional compound intended for preventing or treating attention deficit and/or hyperactivity and/or impulsivity.

The present invention also relates to a pharmaceutical composition comprising as active substances, at least one compound of formula (I) as defined above, or a pharmaceutically acceptable salt thereof, and at least one additional compound intended for preventing or treating attention deficit and/or hyperactivity and/or impulsivity, in particular selected from the group constituted of methylphenidate, dextroamphetamine, dextroamphetamine-amphetamine, lisdexamfetamine, atomoxetine, bupropion, desipramine, clonidine and guanfacine, optionally in association with a pharmaceutically acceptable vehicle.

The present invention also relates to products containing:
- at least one compound of formula (I) as defined above, or a pharmaceutically acceptable salt thereof, and
- at least one additional compound intended for preventing or treating attention deficit and/or hyperactivity and/or impulsivity,
as a combined preparation for simultaneous, separate or sequential use in the prevention or treatment of attention deficit and/or hyperactivity and/or impulsivity in an individual.

### Description of the invention

Preferably, the above-defined compound of formula (I) is represented by the following formula (II): 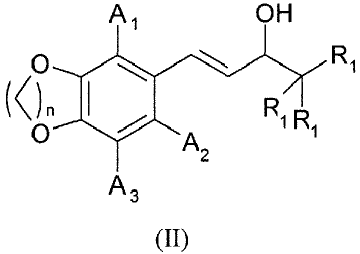 in which n, A₁, A₂, A₃ and R₁ are as defined above.

More preferably the above-defined compound of formula (I) or (II) is represented by the following formula (III):

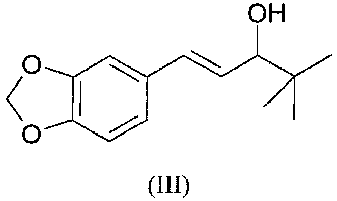

Preferred alkyl groups according to the invention encompass the methyl, ethyl, n-propyl, isopropyl, n-butyl, s-butyl and t-butyl groups. The Cl, I, Br or F atoms are preferred halogen atoms according to the invention.

French patent FR 2 173 691, which is incorporated herein by reference, describes the synthesis of Stiripentol, in particular starting from methylenedioxy-3,4-phenyl)-1-dimethyl-4,4-penten-1-on-3. It is well within the ordinary skills of one of skill in the art to synthesize the other compounds of formula (I) from this teaching.

As will be clear to one of skill in the art, the above-defined formulas (I), (II), and (III) represent either the various stereoisomers encompassed by these formulas or mixtures thereof, in particular racemic mixtures thereof.

Thus, the compound of formula (III) can be a compound of formula (IIIa) a compound of formula (IIIb), or a mixture of a compound of formula (IIIa) and a compound of formula (IIIb), in particular the racemic mixture thereof.

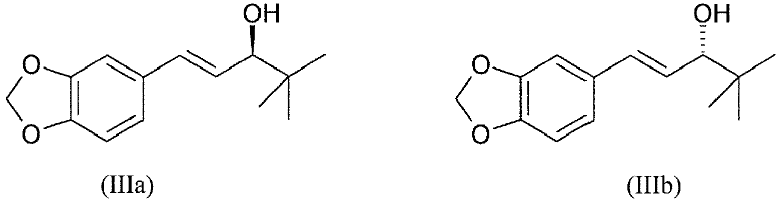

Hyperactivity, impulsivity, and attention deficit, which may also be referred to as inattention, are well known to one of skill in the art.

By way of example, attention deficit of an individual, in particular a child, can be evidenced by one or more of the following: short duration of spontaneous activities, often leaving play activities unfinished, over-frequent changes between activities, undue lack of persistence at tasks set by adults, or unduly high distractibility during study e.g. homework or reading assignment.

By way of example, hyperactivity of an individual, in particular a child, can be evidenced by one or more of the following: very often runs about or climbs excessively in situations where it is inappropriate, seems unable to remain still, markedly excessive fidgeting and wriggling during spontaneous activities, markedly excessive activity in situations expecting relative stillness (e.g. mealtimes, travel, visiting, church), often leaves seat in classroom or other situations when remaining seated is expected, or often has difficulty playing quietly.

By way of example, impulsivity of an individual, in particular a child, can be evidenced by one or more of the following: often has difficulty awaiting turns in games or group situations, often interrupts or intrudes on others (e.g. butts in to others' conversations or games), or often blurts out answers to questions before questions have been completed.

Preferably, attention deficit hyperactivity disorder (ADHD) or hyperkinetic disorder has to be prevented or treated in the above-defined individual.

Attention deficit hyperactivity disorder and hyperkinetic disorder are well known to one of skill in the art.

By way of example, the fifth edition of the Diagnostic and Statistical Manual of Mental Disorders (DSM-5) (American Psychiatric Publishing, 2013) defines the following criteria for ADHD:
People with ADHD show a persistent pattern of inattention and/or hyperactivity-impulsivity that interferes with functioning or development:
   1. Inattention: Six (or more) of the following symptoms have persisted for at least 6 months to a degree that is inconsistent with developmental level and that negatively impacts directly on social and academic/occupational activities. Note: the symptoms are not solely a manifestation of oppositional behavior, defiance, hostility, or failure to understand tasks or instructions. For older adolescents and adults (age 17 and older), at least five symptoms are required.
      o Often fails to give close attention to details or makes careless mistakes in schoolwork, at work, or with other activities (e.g.: overlooks or misses details, work is inaccurate).
      o Often has difficulty sustaining attention in tasks or play activities (e.g.: has difficulty remaining focused during lectures, conversations, or lengthy reading).
      o Often does not seem to listen when spoken to directly (e.g.: mind seems elsewhere, even in the absence of any obvious distraction).
      o Often does not follow through on instructions and fails to finish schoolwork, chores, or duties in the workplace (e.g., starts tasks but quickly loses focus and is easily sidetracked).
      o Often has difficulty organizing tasks and activities (e.g.: difficulty managing sequential tasks; difficulty keeping materials and belongings in order; messy, disorganized work; has poor time management; fails to meet deadlines).
      o Often avoids, dislikes, or is reluctant to engage in tasks that require sustained mental effort (e.g.: schoolwork or homework; for older adolescents and adults, preparing reports, completing forms, reviewing lengthy papers).
      o Often loses things necessary for tasks and activities (e.g. school materials, pencils, books, tools, wallets, keys, paperwork, eyeglasses, mobile telephones).
      o Is often easily distracted by extraneous stimuli (for older adolescents and adults, may include unrelated thoughts).
      o Is often forgetful in daily activities (e.g.: doing chores, running errands; for older adolescents and adults, returning calls, paying bills, keeping appointments).
   2. Hyperactivity and Impulsivity: Six (or more) of the following symptoms have persisted for at least 6 months to a degree that is inconsistent with developmental level and that negatively impacts directly on social and academic/occupational activities. Note: the symptoms are not solely a manifestation of oppositional behavior, defiance, hostility, or failure to understand tasks or instructions. For older adolescents and adults (age 17 and older), at least five symptoms are required.
      o Often fidgets with or taps hands or feet, or squirms in seat.
      o Often leaves seat in situations when remaining seated is expected (e.g.: leaves his or her place in the classroom, in the office or other workplace, or in other situations that require remaining in place).
      o Often runs about or climbs in situations where it is not appropriate (Note: in adolescents or adults, may be limited to feeling restless).
      o Often unable to play or engage in leisure activities quietly.
      o Is often "on the go" acting as if "driven by a motor" (e.g.: is unable to be or uncomfortable being still for extended time, as in restaurants, meetings; may be experienced by others as being restless or difficult to keep up with).
      o Often talks excessively.
      o Often blurts out an answer before a question has been completed (e.g.: completes people's sentences; cannot wait for turn in conversation).
      o Often has difficulty waiting his/her turn (e.g.: while waiting in line).
      o Often interrupts or intrudes on others (e.g., butts into conversations, games or activities; may start using other people's things without asking or receiving permission; for adolescents and adults, may intrude into or take over what others are doing).
In addition, the following conditions must be met:
- Several inattentive or hyperactive-impulsive symptoms were present prior to age 12 years.
- Several inattentive or hyperactive-impulsive symptoms are present in two or more setting, (e.g., at home, school or work; with friends or relatives; in other activities).
- There is clear evidence that the symptoms interfere with, or reduce the quality of, social, academic, or occupational functioning.
- The symptoms do not occur exclusively during the course of schizophrenia or another psychotic disorder and are not better explained by another mental disorder (*e.g*. Mood Disorder, Anxiety Disorder, Dissociative Disorder, Personality Disorder, substance intoxication or withdrawal).

Thus, based on the types of symptoms, three presentations of ADHD can be specified:
*Combined Presentation:* if both Criterion 1 (inattention) and Criterion 2 (hyperactivity-impulsivity) are met for the past 6 months.
*Predominantly Inattentive Presentation:* if Criterion 1 (inattention) is met but Criterion 2 (hyperactivity-impulsivity) is not met for the past six months
*Predominantly Hyperactive-Impulsive Presentation:* if Criterion 2 (hyperactivity-impulsivity) is met and Criterion 1 (inattention) is not met for the past six months. Because symptoms can change over time, the presentation may change over time as well.

By way of example, the tenth version of the international classification of diseases (ICD-10) established by the World Health Organization (WHO) regarding the classification of mental and behavioural disorders defines the following criteria for hyperkinetic disorder:
Note: The research diagnosis of hyperkinetic disorder requires the definite presence of abnormal levels of inattention and restlessness that are pervasive across situations and persistent over time, that can be demonstrated by direct observation, and that are not caused by other disorders such as autism or affective disorders.
Eventually, assessment instruments should develop to the point where it is possible to take a quantitative cut-off score on reliable valid and standardized measures of hyperactive behavior in the home and classroom, corresponding to the 95th percentile on both measures. Such criteria would then replace G1 and G2 below.

**G1.** Demonstrable abnormality of attention, activity and impulsivity at home, for the age and developmental level of the child, as evidenced by (1), (2) and (3):
   (1) at least three of the following attention problems:
      (a) short duration of spontaneous activities;
      (b) often leaving play activities unfinished;
      (c) over-frequent changes between activities;
      (d) undue lack of persistence at tasks set by adults;
      (e) unduly high distractibility during study e.g. homework or reading assignment;
   (2) plus at least three of the following activity problems:
      (a) very often runs about or climbs excessively in situations where it is inappropriate; seems unable to remain still;
      (b) markedly excessive fidgeting & wriggling during spontaneous activities;
      (c) markedly excessive activity in situations expecting relative stillness (e.g. mealtimes, travel, visiting, church);
      (d) often leaves seat in classroom or other situations when remaining seated is expected;
      (e) often has difficulty playing quietly.
   (3) plus at least one of the following impulsivity problems:
      (a) often has difficulty awaiting turns in games or group situations;
      (b) often interrupts or intrudes on others (e.g. butts in to others' conversations or games);
      (c) often blurts out answers to questions before questions have been completed.
**G2.** Demonstrable abnormality of attention and activity at school or nursery (if applicable), for the age and developmental level of the child, as evidenced by both (1) and (2):
   (1) at least two of the following attention problems:
      (a) undue lack of persistence at tasks;
      (b) unduly high distractibility, i.e. often orienting towards extrinsic stimuli;
      (c) over-frequent changes between activities when choice is allowed;
      (d) excessively short duration of play activities;
   (2) and by at least three of the following activity problems:
      (a) continuous (or almost continuous) and excessive motor restlessness (running, jumping, etc.) in situations allowing free activity;
      (b) markedly excessive fidgeting and wriggling in structured situations;
      (c) excessive levels of off-task activity during tasks;
      (d) unduly often out of seat when required to be sitting;
      (e) often has difficulty playing quietly.
**G3.** Directly observed abnormality of attention or activity. This must be excessive for the child's age and developmental level. The evidence may be any of the following:
   (1) direct observation of the criteria in G1 or G2 above, *i.e.* not solely the report of parent or teacher;
   (2) observation of abnormal levels of motor activity, or off-task behavior, or lack of persistence in activities, in a setting outside home or school (*e.g*. clinic or laboratory);
   (3) significant impairment of performance on psychometric tests of attention.
**G4.** Does not meet criteria for pervasive developmental disorder (F84), mania (F30), depressive (F32) or anxiety disorder (F41).
**G5.** Onset before the age of seven years.
**G6.** Duration of at least six months.
**G7.** IQ above 50.

The individual according to the invention is preferably a human, more preferably a child, in particular under 18, 17, 16, 15, 14, 13, 12, 11, 10, 9, 8, 7, 6, 5, or 4 years of age. Besides, in an embodiment of the invention, the individual according to the invention does not suffer from epilepsy.

Preferably, the compound of formula (I) as defined above or the pharmaceutically acceptable salt thereof, is to be administered at a unit dose of from 100 mg to 1000 mg or of from 5 mg/kg to 100 mg/kg. Preferably also, the compound of formula (I) as defined above or the pharmaceutically acceptable salt thereof is to be administered with a dosage regimen of from 10 mg/kg/d to 200 mg/kg/d.

Preferably, the compound of formula (I) as defined above or the pharmaceutically acceptable salt thereof, the pharmaceutical composition for use as defined above, the pharmaceutical composition as defined above or the medicament as defined above, is in a form suitable for administration by the oral or rectal route. Preferably also, the compound of formula (I) as defined above or the pharmaceutically acceptable salt thereof, the pharmaceutical composition as defined above or the medicament as defined above, is in the form of a powder, sachets, tablets, capsules or suppositories.

As intended herein, the expression "additional compound intended for preventing or treating attention deficit and/or hyperactivity and/or impulsivity" relates to any compound intended to alleviate one or more of the symptoms of or to treat or prevent attention deficit and/or hyperactivity and/or impulsivity, in particular attention deficit hyperactive disorder (ADHD) and/or hyperkinetic disorder. Preferably, the at least one additional compound intended for preventing or treating attention deficit and/or hyperactivity and/or impulsivity is selected from the group constituted of a stimulant such as methylphenidate, dextroamphetamine (also called dexamphetamine or dexamfetamine), dextroamphetamine-amphetamine *(i.e.* a combined preparation of dextramphetamine and amphetamine (also called amfetamine)), or lisdexamfetamine, atomoxetine, and an antidepressant such as bupropion, desipramine, clonidine or guanfacine.

As intended herein "combined" or "in combination" means that the compound of formula (I) as defined above or the pharmaceutically acceptable salt thereof, in particular Stiripentol, is administered at the same time than the additional compound, either together, i.e. at the same administration site, or separately, or at different times, provided that the time period during which the compound of formula (I) as defined above or the pharmaceutically acceptable salt thereof exerts its pharmacological effects on the individual and the time period during which the additional compound exerts its pharmacological effects on the individual, at least partially intersect.

### Description of the figures

### Figure 1

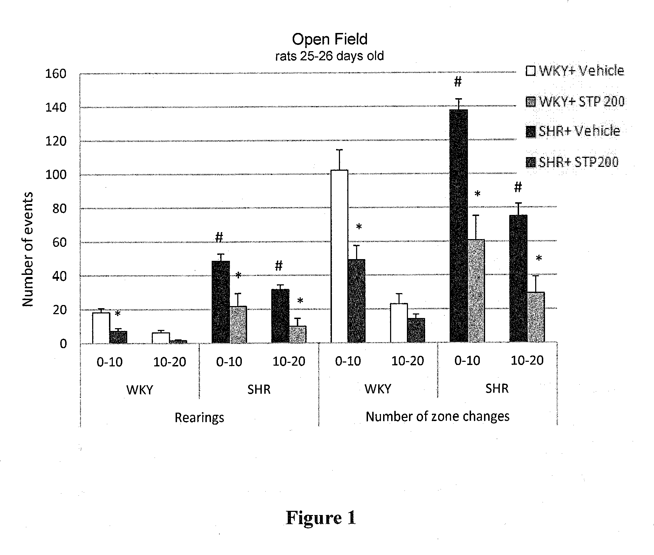

Figure 1 represents the effect of vehicle (veh, white bars, black bars) or Stiripentol at 200 mg/kg (STP 200, gray bars) treatment on the number of events (mean ± SEM) relating to rearings (left) or zone changes (right) of WKY (control) or SHR juveniles rats (25-26 days old) in open field during the first 10 minutes (0-10) of the introduction of the rats in the arena and the next 10 minutes (10-20).
# : p <0.001, SHR compared to WKY rats, two way analysis of variance, followed by a post-hoc Holm-Sidak procedure.
* : p <0.001, STP- treated compared to vehicle-treated rats, two way analysis of variance, followed by a post-hoc Holm-Sidak procedure.

### Figure 2

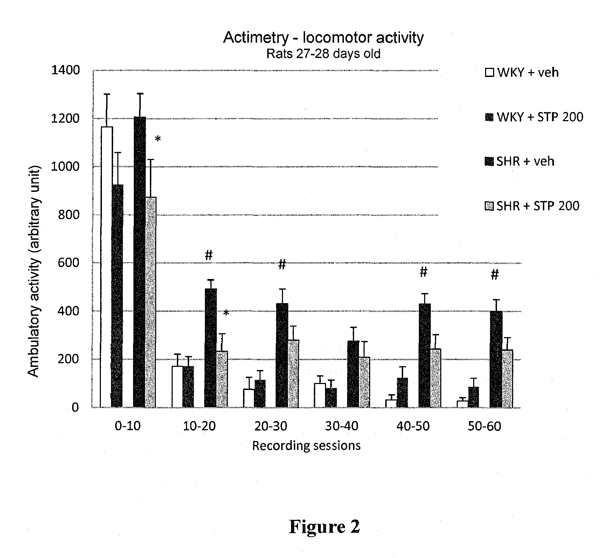

Figure 2 represents the effect of (veh, white bars, black bars) or Stiripentol at 200 mg/kg (STP 200, gray bars) treatment on ambulatory (or locomotor) activity (mean ± SEM arbitrary units) of WKY (control) or SHR juveniles rats (27-28 days old) measured in actimetry during the first 10-minutes recording session (0-10) of the introduction of the rats in the in the opto-varimex system and the next 10-minutes recording sessions (10-20, 20-30, 30-40, 40-50 and 50-60).
#: p <0.001, SHR compared to WKY rats, two way repeated measures analysis of variance, followed by a post-hoc Holm-Sidak procedure.
*: p <0.001, STP-treated rats compared to vehicle rats, two way repeated measures analysis of variance, followed by a post-hoc Holm-Sidak procedure.

### Figure 3

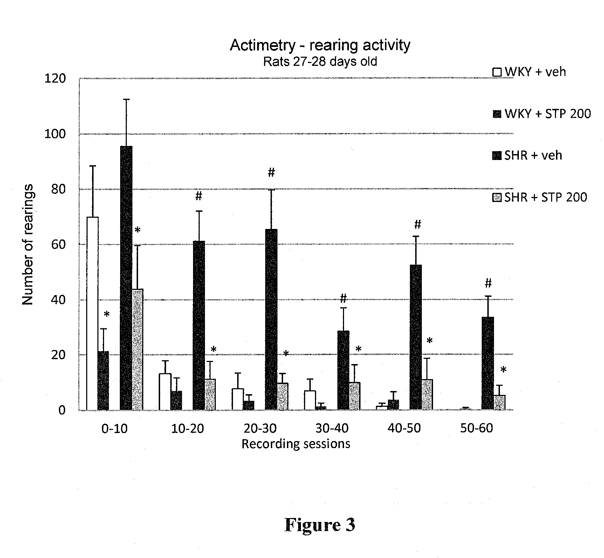

Figure 3 represents the effect of (veh, white bars, black bars) or Stiripentol at 200 mg/kg (STP 200, gray bars) treatment on the number of rearings (rearing activity) (mean ± SEM arbitrary units) of WKY (control) or SHR juveniles rats (27-28 days old) measured in actimetry during the first 10-minutes recording session (0-10) of the introduction of the rats in the in the opto-varimex system and the next 10-minutes recording sessions (10-20, 20-30, 30-40, 40-50 and 50-60).
#: p <0.001, SHR compared to WKY rats, two way analysis of variance, followed by a post-hoc Holm-Sidak procedure.
*: p <0.001, STP-treated rats compared to veh rats, two way analysis of variance, followed by a post-hoc Holm-Sidak procedure.

### Figure 4

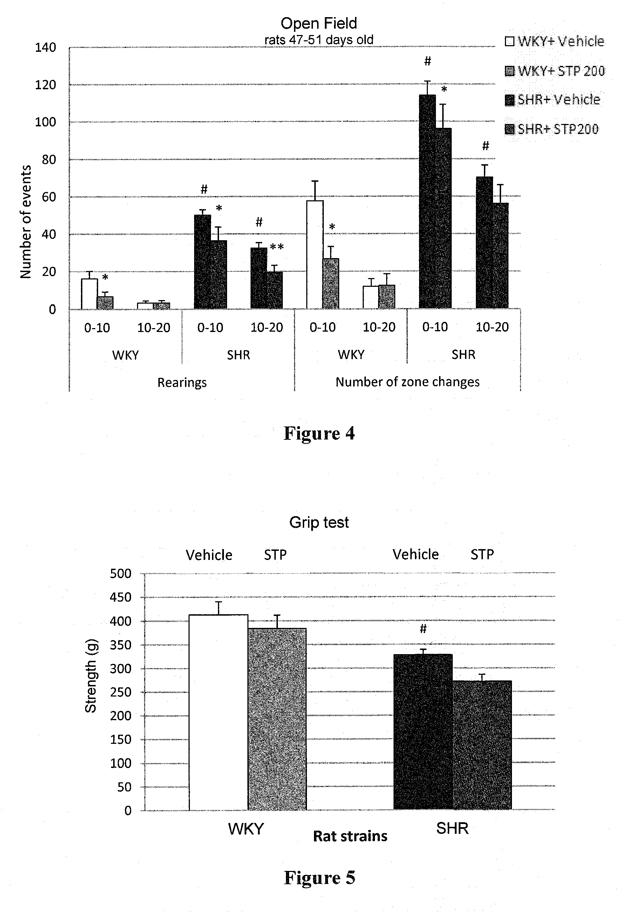

Figure 4 represents the effect of vehicle (white bars, black bars) or Stiripentol at 200 mg/kg (STP 200, gray bars) treatment on the number of events (mean ± SEM) relating to rearings (left) or zone changes (right) of WKY (control) or SHR adolescent rats (47-51 days old) in open field during the first 10 minutes (0-10) of the introduction of the rats in the arena and the next 10 minutes (10-20).
# : p <0.001, SHR compared to WKY rats, two way analysis of variance, followed by a post-hoc Holm-Sidak procedure.
*: p <0.05, **: P<0.01 STP-treated rats compared to vehicle rats, two way analysis of variance, followed by a post-hoc Holm-Sidak procedure.

### Figure 5

Figure 5 represents the effect of vehicle (white bars, black bars) or Stiripentol at 200 mg/kg (STP, gray bars) treatment on the strength (in g, mean ± SEM) of WKY (control) or SHR adolescent rats (40-45 days old).
# : p <0.001, SHR compared to WKY rats, two way analysis of variance, followed by a post-hoc Holm-Sidak.

### EXAMPLE

The inventors have examined the potential new therapeutic application of Stiripentol (STP, Diacomit®) in attention deficit and hyperactivity disorder (ADHD). For this, the effect of an acute administration of Stiripentol, on hyperactivity in rats was studied. The model chosen for this study is the Spontaneously Hypertensive Rat (SHR), a rat model that has been described as a model for hyperactivity (Sagvolden et al. (1992) Behav. Neural. Biol. 58:103-112; Sagvolden et al. (2009) Neuropharmacology 57:619-626; Davids et al. (2003) Brain Res. Brain Res. Rev. 42:1-21) and three behavioral tests were run: an open field test, an actimetry test and a grip test.

### 1 MATERIALS AND METHODS

### 1.1 ANIMALS

Juvenile (25-30 days old, 80-120g) or adolescent (40-45 days old, 140-160g) male Wistar SHR rats or the control strain Wistar kyoto (WKY) rats, from River Germany, were used in this study. They were housed 2 per cage (Techniplast ref. 1291), and maintained in a 12h light/dark cycle (light ON at 7 AM). Food and water were provided *ad libitum.* Rats were acclimated to the animal facilities at least 5 days before testing started. The experiments were conducted in accordance with the European Recommendations (directive 86/609/EEC) for the use and care of laboratory animals.

### 1.2 BEHAVIORAL TESTING

### Open field test:

Rats are placed in the middle of an open field (a closed arena of 1m x 1m), and recorded with a videotracking system (Videotrack, Viewpoint S.A., Lyon France). The arena is separated in 9 equivalent zones on the analysis software. Illumination intensity is set around 30 lux. The number of rearings and zone changes is recorded during 2 consecutive 10 minutes periods. The test is started forty five minutes after vehicle or STP injection. For juvenile rats (around 25 days old, n=30 SHR rats, n=30 WKY rats), each rat was randomly assigned to a test group and tested once. For studies with adolescent rats (45 days old, n=10 SHR rats and n=10 WKY rats), a counterbalanced design was used, with a 48 hours delay between the two injections.

### Actimetry:

Animals are placed in the opto-varimex system (Plexiglas arena, 42 x 42 cm, Columbus Instruments, Columbus, Ohio), an automated system to quantify their activity as horizontal or vertical movements. Animals are habituated to the behavioral room for one hour before the beginning of the test, illumination intensity is around 400 lux. Activity is recorded immediately after placing the animal in the system, forty five minutes after injection, for a one hour period. Animals are assigned to a test group and tested only once.

### Grip test:

The grip test measures the muscular strength of the animal. This test is used to identify myorelaxant properties of compounds. An electronic system is used (Bioseb) to measure the force developed by a rat grasping a T-shape bar with its forelimb. Five consecutive measures are taken, data are expressed as mean ± standard error of mean. Rats (45-50 days old, n=10 SHR rats and n=10 WKY rats) are tested following a counterbalanced design, with a 48 hours delay between the two injections (vehicle or STP 200 mg/kg). Measures are taken 60 minutes after intraperitoneal (i.p.) injection.

### 1.3 PHARMACOLOGICAL TREATMENT

Stiripentol (batch 176, dissolved in 5% tween 80 in 0.9% NaCl) was administered i.p. at the dose of 200 mg/kg with an injection volume of 10 ml/kg.

### 1.4 DATA ANALYSIS

Data are represented as the mean ± standard error of mean (S.E.M.). The difference between Stiripentol and vehicle treated rats, was assessed with an ANOVA or repeated measures ANOVA (one factor repetition). Significance was set at p<0.05 (Sigma Stat, v3.5, SPSS, Chicago, USA).

### 2 RESULTS

### 2.1 EFFECT OF STIRIPENTOL (STP) ADMINISTRATION IN THE OPEN FIELD-JUVENILE RATS (25 DAYS OLD)

In the open field test, a statistically significant difference is found between WKY and SHR rats (#, p< 0.001, ANOVA), for both variables studied (rearings and number of zone changes) **(****Figure 1**).

It should be noted that control WKY rats present a high level of activity during the first 10 minutes period due to their exploration of previously unknown arena before getting accustomed to it for the second 10 minutes period. Therefore, only the second period of testing truly reflects the hyperactive behavior of rats.

During this second period of testing, Stiripentol treatment significantly decreases rearing activity in the SHR rats at the dose of 200 mg/kg (p< 0.001, ANOVA, Holm-Sidak post-hoc test), but has no effect on the control WKY rats.

Similarly, the number of zone changes was decreased after STP treatment in SHR rats only (p< 0.001, ANOVA, Holm-Sidak post-hoc test).

### 2.2 EFFECT OF STIRIPENTOL (STP) ON RATS ACTIVITY JUVENILE RATS

In the actimetry test, two variables were studied: number of horizontal movements and number of rearings. Two-way repeated measures ANOVAs showed a significant interaction between strain and time (p = 0.009) and between strain and treatment (p = 0.002) for the analysis of the variable "ambulatory activity" **(****Figure 2****)**. Significant interaction between strain and treatment was p < 0.001 for the variable "rearing" **(****Figure 3****)**.

The difference between the two strains WKY and SHR was statistically significant for both variables studied (horizontal movements and rearing), for all recording sessions studied, except for the first time period of 0-10 minutes for both variables, and 30-40 minutes for the variable "ambulatory activity" (Two way repeated measures ANOVA, post-hoc Holm-Sidak).

In the WKY control group, there was no effect of treatment with STP on rats' locomotor activity. Whereas, in the SHR group, there is a statistically significant difference in the ambulatory activity between Stiripentol-treated rats and vehicle-treated rats (repeated measures ANOVA, post-hoc Holm-Sidak).

Likewise, Stiripentol has no effect on the number of rearings in the WKY group, whereas treatment with Stiripentol statistically decreases the rearing behavior in the SHR group (repeated measures ANOVA, post-hoc Holm-Sidak) **(****Figure 3****)**.

### 2.3 EFFECT OF STIRIPENTOL (STP) OR VEHICULE IN OPEN FIELD - ADOLESCENT RA TS (47-51 DA YS OLD)

For the analysis of the dependent variable "rearing" in the open field, the two way analysis of variance showed a statistically significant difference between the two strains, WKY and SHR, and between treatments: vehicle or STP in the second 10 minutes recording session **(****Figure 4****).**

For the analysis of the other variable "number of zones change", the two way analysis of variance showed a statistically significant difference between the two strains, WKY and SHR.

### 2.4 EFFECT OF STIRIPENTOL OR VEHICULE IN THE GRIP TEST( 40-45 DAYS OLD RATS)

Strength developed in the grip test was measured 1 hour after vehicle or STP 200 mg/kg administration **(****Figure 5****)**.

Results show a statistically significant difference between WKY and SHR strains (p<0.001, ANOVA). There is no statistically significant interaction between strains and treatment (P = 0.544) and no significant effect of treatment (p= 0.058).

### 3 DISCUSSION AND CONCLUSION

The present results have shown strains differences between WKY and SHR rats in the different behavioral tests, and therefore validated this model of hyperactivity.

In addition, Stiripentol treatment generally induced a decrease of activity specifically in the hyperactive rat (SHR) with respect to the non hyperactive control rats (WKY).

The grip test indicated that there was a difference between SHR and control rats in the strength developed by the forelimbs. A difference in muscle fibers types has been described between SHR and control WKY rats (Nagatomo et al. (2009) J. Atheroscler. Thromb. 16:576-585). However, there was no effect of STP in either strain, which shows that the effect of STP is not related to a myorelaxant effect.

## Claims

1. A compound of the following formula (I): 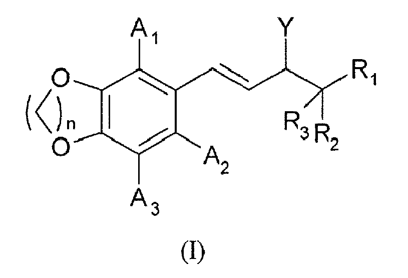 wherein:
- n represents 1 or 2,
- A₁, A₂ and A₃, which may be identical or different, represent a hydrogen atom, a halogen atom or a linear or branched alkyl group having from 1 to 4 carbon atoms,
- R₁, R₂ and R₃ represent independently a hydrogen atom or a linear or branched alkyl group having from 1 to 4 carbon atoms, and
- Y represents -OH, =O or -SH;
or a pharmaceutically acceptable salt thereof,
for use in the prevention or treatment of attention deficit and/or hyperactivity and/or impulsivity in an individual.

2. The compound or pharmaceutically acceptable salt thereof for use according to claim 1, wherein the compound or pharmaceutically acceptable salt thereof is of the following formula (II): 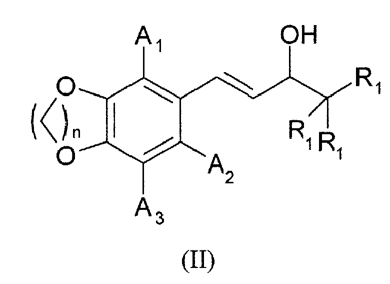 wherein n, A₁, A₂, A₃ and R₁ are as defined in claim 1.

3. The compound or pharmaceutically acceptable salt thereof for use according to claim 1 or 2, wherein the compound or pharmaceutically acceptable salt thereof is of the following formula (III):

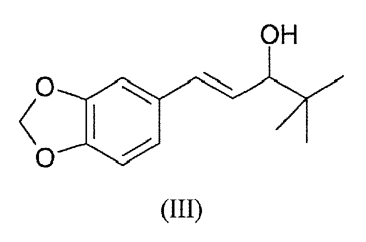

4. The compound or pharmaceutically acceptable salt thereof for use according to any of claims 1 to 3, in the prevention or treatment of attention deficit hyperactivity disorder (ADHD) or hyperkinetic disorder.

5. The compound or pharmaceutically acceptable salt thereof for use according to any of claims 1 to 4, wherein the compound or pharmaceutically acceptable salt thereof is administered at a unit dose of from 5 mg/kg to 100 mg/kg or of from 100 mg to 1000 mg.

6. The compound or pharmaceutically acceptable salt thereof for use according to any of claim 1 to 5, wherein the compound or pharmaceutically acceptable salt thereof is administered at a dose regimen of from 10 mg/kg/d to 200 mg/kg/d.

7. The compound or pharmaceutically acceptable salt thereof for use according to any of claims 1 to 6, wherein the compound or pharmaceutically acceptable salt thereof is in a form suitable for administration by the oral or rectal route.

8. The compound or pharmaceutically acceptable salt thereof for use according to any of claims 1 to 7, wherein the compound or pharmaceutically acceptable salt thereof is in the form of a powder, sachets, tablets, capsules or suppositories.

9. The compound or pharmaceutically acceptable salt thereof according to any of claims 1 to 8, in combination with at least one additional compound intended for preventing or treating attention deficit and/or hyperactivity and/or impulsivity.

10. The compound or pharmaceutically acceptable salt thereof according to claim 9, wherein the at least one additional compound intended for preventing or treating attention deficit and/or hyperactivity and/or impulsivity is selected from the group constituted of methylphenidate, dextroamphetamine, dextroamphetamine-amphetamine, lisdexamfetamine, atomoxetine, bupropion, desipramine, clonidine and guanfacine.

11. A pharmaceutical composition, comprising as active substances at least one compound of formula (I) as defined in any of claims 1 to 3, or a pharmaceutically acceptable salt thereof, for use in the prevention or treatment of attention deficit and/or hyperactivity and/or impulsivity in an individual.

12. The pharmaceutical composition for use according to claim 11, in the prevention or treatment of attention deficit hyperactivity disorder (ADHD) or hyperkinetic disorder.

13. The pharmaceutical composition for use according to claim 11 or 12, further comprising at least one additional compound intended for preventing or treating attention deficit and/or hyperactivity and/or impulsivity.

14. The pharmaceutical composition for use according to claim 13, wherein the at least one additional compound intended for preventing or treating attention deficit and/or hyperactivity and/or impulsivity selected from the group constituted of methylphenidate, dextroamphetamine, dextroamphetamine-amphetamine, lisdexamfetamine, atomoxetine, bupropion, desipramine, clonidine and guanfacine.

15. A pharmaceutical composition comprising as active substances, at least one compound of formula (I) as defined in claims 1 to 3, or a pharmaceutically acceptable salt thereof, and at least one additional compound intended for preventing or treating attention deficit and/or hyperactivity and/or impulsivity selected from the group constituted of methylphenidate, dextroamphetamine, dextroamphetamine-amphetamine, lisdexamfetamine, atomoxetine, bupropion, desipramine, clonidine and guanfacine, optionally in association with a pharmaceutically acceptable vehicle.

16. Products containing:
- at least one compound of formula (I) as defined in claims 1 to 3, or a pharmaceutically acceptable salt thereof, and
- at least one additional compound intended for preventing or treating attention deficit and/or hyperactivity and/or impulsivity as defined in claim 9 or 10,
as a combined preparation for simultaneous, separate or sequential use in the prevention or treatment of attention deficit and/or hyperactivity and/or impulsivity in an individual.

17. Products according to claim 16, in the prevention or treatment of attention deficit hyperactivity disorder (ADHD) or hyperkinetic disorder.
